# EUROPÄISCHE PATENTSCHRIFT

(11) **EP 1 322 305 B1**
(45) Veröffentlichungstag und Bekanntmachung des Hinweises auf die Patenterteilung: **07.12.2005**
(21) Anmeldenummer: 01983497.7
(22) Anmeldetag: 24.09.2001
(51) Int. Cl.: A61K 31/19, A61P 19/02

(54) **VERWENDUNG VON R-ARYLPROPIONSÄUREN ZUR HERSTELLUNG VON ARZNEIMITTELN ZUR BEHANDLUNG VON ERKRANKUNGEN RHEUMATISCHER NATUR**
USE OF R-ARYLPROPIONIC ACIDS FOR PRODUCING MEDICAMENTS FOR TREATING ILLNESSES WITH A RHEUMATIC NATURE
UTILISATION D'ACIDES R-ARYLPROPIONIQUES DANS LA PRODUCTION DE MEDICAMENTS DESTINES A TRAITER DES MALADIES DE NATURE RHUMATISMALE

(30) Priorität: 25.09.2000 DE 10047319
(43) Veröffentlichungstag der Anmeldung: 02.07.2003
(73) Patentinhaber: PAZ Arzneimittel- Entwicklungsgesellschaft mbH, 65933 Frankfurt am Main (DE)
(72) Erfinder: GEISSLINGER, Gerd, 65812 Bad Soden (DE); GRÖSCH, Sabine, 65321 Heidenrod-Egenroth (DE)
(74) Vertreter: Wagner, Jutta
(86) Internationale Anmeldenummer: PCT/EP2001/011004
(87) Internationale Veröffentlichungsnummer: WO 2002/024190

(56) Entgegenhaltungen:
- WO-A-00/13684
- WO-A-00/50019
- WO-A-94/20449
- DE-A- 4 140 183
- DE-A- 4 140 184
- US-A- 5 200 198

## Beschreibung

Gegenstand der vorliegenden Erfindung ist die Verwendung von R-Arylpropionsäuren zur Herstellung von Arzneimitteln zur Behandlung von Erkrankungen, welche durch die Hemmung der Aktivierung des nukteären Transkriptionsfaktors AP-1 therapeutisch beeinflußt werden können, insbesondere Erkrankungen rheumatischer Natur.

Unter den Begriff Rheuma fallen mehrere hundert Erkrankungen im Bereich des Bewegungsapparates (z. B. Gelenke, Gelenkkapseln, Knochen, Muskulatur oder Sehnen) oder der Bindegewebsstrukturen (fast überall im Körper vorhanden), welche sich durch schmerzhafte oder entzündliche Prozesse auszeichnen. Vor allem bei chronischen Verlaufsformen von Gelenkerkrankungen durch Verschleiß (Arthrosen) oder Gelenkerkrankungen durch Entzündung (Arthritis) können der Knorpel und der Knochen und im schlimmsten Falle auch die Organe angegriffen werden.

Die Behandlung rheumatischer Erkrankungen richtet sich beim einzelnen Patienten nach der Schwere und der Aggressivität der Krankheit Neben anderen therapeutischen Maßnahmen hat die medikamentöse Rheumatherapie einen hohen Stellenwert. Abhängig vom Krankheitszustand werden Medikamente aus 5 Hauptgruppen eingesetzt:
1. Reine Schmerzmittel: z. B. Paracetamol, Metamizol, Tramadol
2. Nicht-steroidale Entzündungshemmer (NSAR/NSAID): z. B. Diclofenac, Ibuprofen, Flurbiprofen, Naproxen, Indometacin, Piroxicam, Meloxicam sowie neuere COX-2 Inhibitoren, z. B. Celecoxib, Rofecoxib
3. Corticosteroide: z. B. Cortison, Prednison, Prednisolon, Dexamethason
4. Langwirksame Antirheumatika (sogenannte "Basismedikamente"): z. B. Goldsalze, Methotrexat, Sulfasalazin, Chloroquin, Ciclosporin
5. Modifikatoren der biologischen Reaktionen ("biologische Therapien"): z. B. die in einigen Ländern neu zugelassenen Substanzen Leflunomid und Etanercept

Ideale Rheumamittel, die einerseits rasch die Schmerz- und Entzündungssymptome mildern und andererseits langfristig die Destruktion von Gelenken verhindern helfen, konnten bisher nicht gefunden werden. Deshalb werden bei akuten Krankheitszuständen eher reine Schmerz- und Entzündungshemmer sowie die stark entzündungshemmenden Corticosteroide eingesetzt. Zur Vermeidung der Langzeitschäden werden zusätzlich die krankheitsmodifizierenden Basistherapeutika eingesetzt. Sie können die Gelenkzerstörung aufhalten, eine Rückbildung der Erkrankung einleiten oder zu einer gewissen Reparatur bereits eingetretener Schäden beitragen.

Sämtliche obengenannten Medikamente (Nr. 1. bis 4.) weisen neben den erwünschten bedeutsame unerwünschte Wirkungen auf. Deshalb ist es oft auch für erfahrene Therapeuten schwierig, für den individuellen Patienten ein optimales Behandlungsschema unter Nutzen-Risiko-Gesichtspunkten festzulegen. Insbesondere der Einsatz der langwirksamen Antirheumatika gestaltet sich als schwierig, weil sie zum Teil erst nach Monaten die erwünschte krankheitsmodifizierende Wirkung zeigen, die unerwünschten Wirkungen jedoch sofort eintreten können. Falls langwirksame Medikamente wie die Goldsalze zu früh abgesetzt werden, nimmt die Wirksamkeit bei einem notwendig werdenden weiteren Einsatz ab. Wegen der Nachteile der bekannten medikamentösen Behandlungsschemata wird mit den neuesten molekularbiologischen Methoden versucht, gezielt in die biochemischen Mechanismen des Entzündungs- und Schmerzgeschehens modulierend einzugreifen. Auf den ersten zur Marktreife entwickelten Substanzen Leflunomid und Etanercept ruhen große Hoffnungen, erwünschte therapeutische Wirkungen ohne die bekannten unerwünschten Wirkungen der anderen Substanzgruppen zu erzielen [Moreland, L. W., et al.: Treatment of rheumatoid arthritis with a recombinat human tumor necrosis factor receptor 8p75-Fc fusion protein. N Engl J Med 337 (1997), 141; Moreland, L. W., et al.: Phase III trial of DMARD failing rheumatoid arthritis patients with TNF receptor p75 Fc fusion protein (TNFR:Fc, ENBREL).J Invest Med 46: 228 A (abstract), 1998]. Umfassende Erfahrungen zu diesen Substanzen liegen noch nicht vor. Der erhoffte therapeutische Fortschritt muß sich erst noch erweisen.

Die Erfindung hat sich zur Aufgabe gestellt, Wirkstoffe zu finden, die in der Lage sind, innerhalb des Krankheitsgeschehens die Gelenkdestruktion aufzuhalten wie die Basistherapeutika (Nr. 4.) ohne die bekannten Hauptnebenwirkungen zu verursachen.

Dieses bei den bisherigen Substanzklassen (Nr. 1 bis 4) nicht zu vereinbarende Eigenschaftsprofil konnte überraschenderweise bei den Substanzen der Arylpropionsäuregruppe gefunden werden, welche bekanntermaßen zur Substanzgruppe der nicht-steroidalen Antirheumatika (Nr. 2) zählen. Substanzen dieser Gruppe sind zum Teil seit mehreren Jahrzehnten als unterschiedlich stark schmerz-, entzündungs- und fieberhemmend wirksam bekannt [Propionic acid derivatives: Goodman & Gilman's, The pharmacological basis of therapeutics, chapter 27, p. 637 (1996)]. Diese Wirkungen werden im wesentlichen der direkten Hemmung der Isoenzyme COX-1 und COX-2 zugeschrieben. Diese Eigenschaften verursachen sowohl die erwünschten, als auch die unerwünschten Wirkungen dieser Substanzen.

Arylpropionsäureverbindungen weisen mit einem asymmetrischen Kohlenstoffatom ein Chiralitätszentrum auf und kommen folglich als S-Enantiomer und als R-Enantiomer vor. Bei der chemischen Synthese fallen diese Wirkstoffe üblicherweise als Racemate, d. h. als gleiche Mischung zwischen den beiden Enantiomeren an. Bis auf S-Naproxen [Williams: Enantiomers in arthritic disorders; Pharmac. Ther., Vol. 46, pp 273 - 295 (1990); Evans: Enantioselective pharmacodynamics und pharmacokinetics of chiral non-steroidal anti-inflammatory drugs. Eur J Clin Pharmacol 42: 237 -256 (1992)] und neuerdings Dexibuprofen [Symposion: Update on S(+)-ibuprofen; Going/Kitzbühel, 2 - 4 February 1996] und Dexketoprofen [First launch of dexketoprofen. Scrip No 2144: 16 (1996)] werden diese Substanzen bislang als Racemate eingesetzt.

Es war bekannt, daß in Bezug auf die COX-Hemmung Unterschiede zwischen den enantiomeren Formen der Arylpropionsäuren bestehen. Die COX-Hemmung wurde nur bei den S-Enantiomeren festgestellt, während sie bei den R-Enantiomeren im therapeutischen Konzentrationsbereich nicht gefunden wurde [Williams (s. o.); Evans (s. o.); Brooks and Day: New nonsteroidal anti-inflammatory drugs, Birkhauser Verlag, Basel S. 119 - 126 (1985)]. Folglich wurden die erwünschten therapeutischen Effekte, aber auch die mit der COX-Hemmung verbundenen unerwünschten Wirkungen den S-Enantiomeren zugeschrieben wie sie in reiner Form oder zur Hälfte im Racemat eingesetzt werden. Bei Substanzen, die im Organismus partiell vom R-Enantiomeren zum S-Enantiomeren invertiert werden, wird eine der Inversionsquote gemäße indirekte Wirkung angenommen [Caldwell et al.: The metabolic chiral inversion and dispositional enantioselectivity of the 2-arylpropionic acids and their biological consequences. Biochemical Pharmacology, Vol 37, No. 1, pp. 105 - 114 (1988)]. Diese Inversion ist bei lbuprofen beim Menschen und allen untersuchten Tierspezies deutlich ausgeprägt. Neben der indirekten Wirkung durch Inversion zu S-Ibuprofen wurde aufgrund von In-vitro-Versuchen eine direkte Wirkung von R-Ibuprofen über eine Hemmung von im Entzündungsgeschehen beteiligten polymorphkernigen Leukozyten spekuliert [Villanueva et al.: Equipotent inhibition by R(-)-, S(+)- and racemic ibuprofen of human polymorphnuclear cell function in vitro. Br. J. Clin. Pharmac., 35, 235 - 242 (1993)]. Eine therapeutische Relevanz dieses Mechanismus konnte bisher nicht gezeigt werden.

Später wurde gefunden, daß auch R-Enantiomere aus der Arylpropionsäuregruppe, z. B. R-Flurbiprofen und R-Ketoprofen eine eigene schmerzlindernde und entzündungshemmende Wirkung zeigen, wenn sie in geeigneter Dosierung bei Mensch oder Tier eingesetzt werden [DE 40 28 906 C2; EP 0 607 128 B1; USA 5,206,029 und 5,200,198; DE 43 19 438 C1; WO 93/17667]. Die Wirkung konnte nicht mit einer direkten COX-Hemmung erklärt werden. In der WO 00/50019 und WO 98/47502 ist beschrieben, daß R-Flurbiprofen bei höheren als den schmerzlindernd wirkenden Konzentrationen im bisher verwendeten Racemat die Aktivierung des transnukleären Transkriptionsfaktors NF-kappa B bei Entzündungsreaktionen hemmt. Wegen der zentralen Stellung von NF-kappa B im Entzündungs- und Immungeschehen konnten aus dieser Entdeckung neue therapeutische Anwendungsmöglichkeiten von R-Flurbiprofen und anderen inversionsstabilen R-Arylpropionsäuren beziehungsweise neue Dosierungsschemata bei Anwendung dieser Substanzen abgeleitet werden. In einer weiteren Patentanmeldung [WO 00/13684] wird die Anwendung von R-Arylpropionsäuren zur Entzündungshemmung beim Tier beansprucht, wobei die Wirkungsweise einer Hemmung der Biosynthese von COX-2 auf der COX-2 mRNA-Ebene zugeschrieben wird. Eine Hemmung der COX-2 Synthese auf dieser Ebene könnte zu einer Hemmung sowohl der induzierbaren COX-2 bei entzündlichen Reaktionen, als auch der konstitutiv vorhandenen (physiologisch im nicht entzündlichen Zustand notwendigen) COX-2 führen. Somit würden Substanzen nach diesem Wirkungsmechanismus auch ohne die direkte COX-2 Hemmung dasselbe Nebenwirkungspotential aufweisen wie die neuen direkten COX-2 Hemmer. In dieser Erfindung wird nicht angegeben, in welchem Ausmaß die Wirkung von R-Arylpropionsäuren auf der Inversion zu den hochwirksamen S-Arylpropionsäuren beruht, die bei den meisten Tierspezies sehr ausgeprägt ist und die bei den hohen angewandten Dosierungen der R-Arylpropionsäuren leicht zu wirksamen Konzentrationen von S-Arylpropionsäuren führen könnte.

Alle bisher bekannt gewordenen Erkenntnisse zur Wirkungsweise der R-Arylpropionsäuren prädestinieren diese Wirkstoffgruppe zur Anwendung als schmerzlindernde und entzündungshemmende Arzneimittel mit kurzer Wirkdauer. Damit sind sie den eingangs beschriebenen Arzneimittelklassen zwischen den reinen Schmerzmitteln (Nr. 1) und den nicht-steroidalen entzündungshemmenden Wirkstoffen (Nr. 2) zuzuorden. Darüber hinaus gehende therapeutische Vorteile waren bisher nicht zu erwarten, da mit der Patentanmeldung WO 00/50019 die therapeutischen Möglichkeiten mit den neuen wissenschaftlichen Erkenntnissen bestens erklärt werden konnten.

Überraschenderweise wurde nun jedoch gefunden, daß Substanzen aus der R-Arylpropionsäuregruppe neben den beschriebenen Kurzzeitwirkungen auch Wirkungen aufweisen, die sie für den Einsatz als krankheitsmodfizierende Arzneimittel geeignet machen. Das heißt, daß sie aufgrund der neu entdeckten Wirkungen in der Lage sind, in den Mechanismus der langfristigen Knochen- und Knorpeldestruktion modulierend einzugreifen. Diese Wirkung wird sonst nur für die langsam und lang wirksamen Basistherapeutika (Nr. 4) angegeben, die jedoch ein wesentlich breiteres Nebenwirkungsspektrum aufweisen.

Die krankheitsmodulierende Wirkung wird verursacht über die erfindungsgemäß festgestellte Hemmung der Aktivierung von Aktivatorprotein 1 (AP-1). Die Hemmung der AP-1 Aktivierung hemmt die Überexpression von Proteinen wie z. B. Metalloproteinasen, Collagenase und Stromelysin, die mit den Stoffwechselprozessen im Gelenk in Zusammenhang gebracht werden. Somit können direkt oder indirekt erosive Prozesse gebremst werden, welche beim Knochenabbau eine Rolle spielen [Handel, M. L. et. al.:Inhibition of transcription factors by anti-inflammatory and anti-rheumatic drugs: Can variability in response be overcome? Clinical and Experimental Pharmacology and Physiology (2000) 27, 139 - 144]. Von einigen Substanzen, z. B. von Dexamethason ist bekannt, daß sie die Aktivierung von NF-kappa B hemmen, was in Einklang mit ihren stark enzündungshemmenden Eigenschaften steht. Die Basistherapeutika Goldthiolat und D-Penicillamin hemmen die AP-1 Aktivierung, was wiederum im Einklang mit der krankheitsmodifizierenden Wirkung steht. Durch die aus der [WO 00/50019] bekannte Entzündungshemmung der R-Arylpropionsäuren über die Hemmung der NF-kappa B Aktivierung in Kombination mit der neu entdeckten krankeitsmodifizierenden Wirkung über die Hemmung der Aktivierung von AP-1 vereint diese Substanzgruppe viele erwünschte Eigenschaften anderer Substanzgruppen ohne deren ungünstiges Nebenwirkungsprofil zu zeigen. Über die Hemmung der Aktivierung von AP-1 kann auch die damit zusammenhängende Überexpression von Zytokinen wie TNF-alpha gehemmt werden. Insofern kann erwartet werden, daß R-Arylpropinsäuren neben den beschriebenen Wirkungen auch ähnliche Wirkungen wie neuen direkten Inhibitoren von TNF-alpha (siehe Nr. 5) aufweisen.

Neben der neugefundenen Wirkung, die eine Langzeitanwendung erfordert, zeigen solche Mittel natürlich weiterhin die bekannten kurzzeitigen Wirkungen der Schmerz- und Entzündungshemmung. Somit üben R-Arylpropionsäuren erstmals eine Doppelfunktion durch raschen Eingriff in das Schmerz- und Entzündungsgeschehen sowie durch Hemmung des erosiv-degenerativ fortschreitenden Gelenkprozesses auf. Anders ausgedrückt, wird mit einem Wirkstoff sowohl symptommodifizierend, als auch krankheits(verlaufs)modifizierend behandelt. Gleichzeitig werden die wesentlichen unerwünschten Wirkungen der ansonsten getrennt anzuwendenden Arzneimittelgruppen vermieden. Da die kurzzeitige entzündungshemmende Wirkung ausreichend hoch dosierter R-Arylpropionsäuren im experimentellen Modell gleich bis besser ausgeprägt ist als bei dem stark wirksamen Cortikosteroid Dexamethason, sind R-Arylpropionsäuren auch ideale Partner für eine Kombinationstherapie zusammen mit Basistherapeutika, auf deren breit belegte Wirksamkeit derzeit nicht verzichtet werden kann. Die Kombinationstherapie zwischen nicht-steroidalen Entzündungshemmem oder Cortikosteroiden und Basistherapeutika ist eines der Standardtherapieverfahren in der Rheumatologie.

Die pharmazeutischen Formulierungen der vorliegenden Erfindung umfassen R-Arylpropionsäure als Wirkstoff oder ein pharmazeutisch verträgliches Derivat davon und einen pharmazeutisch verträglichen Trägerstoff und wahlweise andere therapeutische Zusätze.

Die Ausdrucksweisen "pharmazeutisch verträgliche Derivate" oder "ein pharmazeutisch verträgliches Derivat davon" beziehen sich auf Derivate hergestellt aus pharmazeutisch verträglichen, nichttoxischen Säuren oder Basen, einschließlich anorganischer Säuren und Basen und organischer Säuren und Basen. Da die Komponente der vorliegenden Erfindung sauer ist, können Derivate mit pharmazeutisch verträglichen, nichttoxischen Basen, einschließlich anorganischer und organischer Basen, hergestellt werden. Geeignete pharmazeutisch verträgliche basische Zusatzderivate für die Komponente der vorliegenden Erfindung umfassen Metallsalze, hergestellt aus Aluminium, Calcium, Lithium, Magnesium, Kalium, Natrium und Zink oder organische Salze hergestellt aus Lysin, N,N'-Dibenzylethylendiamin, Cholin, Diethanolamin, Ethylendiamin, Meglumin (N-methylglucamin), Trometamin, Arginin und Alkylaminen mit 1-6 C-Atomen.

Die Formulierungen der vorliegenden Erfindung umfassen Formulierungen wie Suspensionen, Lösungen, Elixiere und Aerosole. Trägerstoffe wie Stärke, Zucker, mikrokristalline Zellulose, Verdünner, Granulierhilfsmittel, Gleitmittel, Bindemittel, Lösemittel und ähnliches können im Falle der festen oralen Applikationsformen verwendet werden. Feste orale Applikationsformen (wie Pulver, Kapseln und Tabletten) werden den flüssigen oralen Applikationsformen vorgezogen. Die bevorzugte feste orale Applikationsform sind Tabletten. Die Tabletten können auf Wunsch mit standardisierten Wasser- oder wasserfreien Beschichtungsmitteln überzogen werden.

Pharmazeutische Formulierungen der vorliegenden Erfindung, welche für die orale Applikationsform geeignet sind, können als separate Einheiten wie Kapseln, Dragees oder Tabletten, oder Aerosole, jeweils eine vorgegebene Menge des Wirkstoffes in Form von Pulver- oder Granulat, oder als Lösung oder Suspension in einer wäßrigen Flüssigkeit, einer nicht wäßrigen Flüssigkeit, einer Öl-in-Wasser Emulsion oder einer flüssigen Wasser-in-Öl Emulsion enthalten. Solche Formulierungen können nach jeder pharmazeutischen Methode hergestellt werden, aber alle Methoden beinhalten eine Vermischung des Wirkstoffes mit einer Trägersubstanz, welche aus einem oder mehreren der notwendigen Bestandteile besteht. Generell werden die Formulierungen durch gleichmäßiges und gründliches Vermischen des Wirkstoffes mit flüssigen Trägersubstanzen oder feinzerkleinerten festen Trägersubstanzen, oder beidem, und dann, falls erforderlich, Formen des Produktes in die gewünschte Applikationsform, hergestellt.

Beispielsweise kann eine Tablette durch Pressen oder Formen, wahlweise mit einem oder mehreren zusätzlichen Bestandteilen hergestellt werden. Gepreßte Tabletten können durch Verpressen in einer entsprechenden Vorrichtung hergestellt werden, wenn der Wirkstoff in einer rieselfähigen Form wie Pulver oder Granulat, wahlweise gemischt mit einem Bindemittel, Gleitmittel, inerten Verdünner, Dispergier- oder oberflächenaktiven Mittel, vorliegt. Geformte Tabletten können auch durch Formen einer Mischung der pulverisierten Komponenten, befeuchtet mit einem inerten flüssigen Verdünner, in einer geeigneten Vorrichtung und anschließender Trocknung hergestellt werden. Vorzugsweise enthält jede Tablette zwischen 30 mg und 1200 mg des Wirkstoffes, und jedes Dragee oder Kapsel enthält zwischen ca. 50 mg und ca. 600 mg des Wirkstoffes. Besonders bevorzugt enthält die Tablette, Dragee oder Kapsel eine von vier Dosierungen, nämlich 50 mg, 100 mg, 200 mg oder 500 mg des Wirkstoffes.

### Beispiel

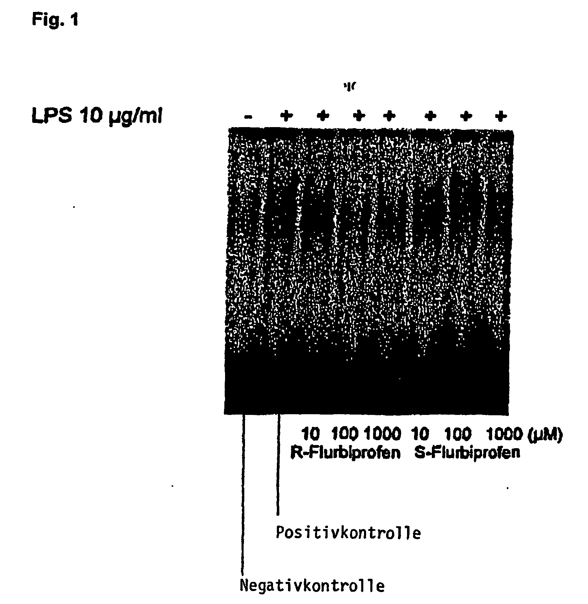

Die Hemmung der Aktivierung von AP-1 wurde mit einem pharmakologischen Modell gezeigt. Dazu wurde eine Zellkultur der murinen Makrophagenzellinie RAW 264.7 angelegt und mit 10 µg/ml LPS für 1 Stunde in Abwesenheit oder in Anwesenheit von Flurbiprofen stimuliert. R-Flurbiprofen (0.1, 1, 10, 100 und 1000 µM) und S-Flurbiprofen (0.1, 1, 10, 100 und 1000 µM) wurden in Phosphatpuffer gelöst den Zellkulturen 30 Minuten vor der LPS-Behandlung zugesetzt. Nach Abschluß der Behandlung wurden die Zellen geerntet und in eine Cytosolfraktion und einen Zellkernextrakt aufgearbeitet. Der Zellkernextrakt (5 - 10 µg) wurde für 30 Minuten mit dem AP-1 spezifischen Oligonukleotid 5'-CGCTTGATGACTCAGCCGGAA-3', welches endständig mit ³²P ATP markiert war, inkubiert. Aus dem Inkubationsmedium wurden die Kem-Protein-DNA Komplexe von der nicht gebundenen DNA durch Elektrophorese (Electrophoretic Mobility Shift Assay: EMSA) getrennt. Nach Trocknung der Gele wurden diese mittels Autoradiographie visualisiert. Typische Ergebnisse sind in Figur 1 dargestellt. Sie zeigen, daß die spezifische AP-1 Bindungsaktivität in LPS-stimulierten Zellen im Vergleich zu unstimulierten Kontrolizellen deutlich zugenommen hat. R-Flurbiprofen (10 - 1000 µM) inhibierte die AP-1 DNA Bindung dosisabhängig. Bei 1000 µM war sie komplett supprimiert. Mit S-Flurbiprofen wurde eine Reduktion nur bei der höchsten Konzentration von 1000 µM gefunden.
- Fig. 1: zeigt die Effekte von R-Flurbiprofen und S-Flurbiprofen auf die DNA-Bindungsaktivität von AP-1 in LPS-stimulierten murinen Macrophagen, bestimmt durch EMSA.

## Patentansprüche

1. Verwendung der R-Enantiomere von Arylpropionsäuren oder deren pharmakologisch verträglichen Salze oder Derivate in reiner oder gegenüber dem Racemat in angereicherter Form zur Herstellung von Arzneimitteln zur Behandlung von chronisch-destruktiven Knorpel- und Gelenkserkrankungen bei Rheuma.

2. Verwendung nach Anspruch 1, **dadurch gekennzeichnet, daß** als R-Arylpropionsäureverbindung R-Flurbiprofen oder dessen Derivate verwendet werden.

3. Verwendung nach Anspruch 1, **dadurch gekennzeichnet, daß** die beim Menschen kaum invertierenden Verbindungen R-Ketoprofen, R-Naproxen, R-Thiaprofensäure oder R-Fenoprofen beziehungsweise pharmakologisch verträgliche Salze oder Derivate dieser Verbindungen eingesetzt werden.

4. Verwendung nach Anspruch 1 bis 3, **dadurch gekennzeichnet, daß** die Wirkstoffe kombiniert mit Basistherapeutika eingesetzt werden.

5. Verwendung nach Anspruch 1 bis 4, **dadurch gekennzeichnet, daß** die Einzeldosierungen der Wirkstoffe zwischen 30 mg und 1200 mg und die Tagesdosierungen zwischen 60 mg und 3600 mg liegen.

6. Verwendung nach Anspruch 1 bis 5, **dadurch gekennzeichnet, daß** die R-Arylpropionsäuren in freier Form, als Salz mit anorganischen oder organischen Salzbildnern beziehungsweise Komplexe, oder als Säureester beziehungsweise Säureamide vorliegen.

7. Verwendung nach Anspruch 1 bis 6, **dadurch gekennzeichnet, daß** die Arzneimittel als pharmazeutische Formulierungen wie Tabletten, Kapseln, Dragees, Trinkgranulate, Trinktabletten, Tropfen, Kauformulierungen, Injektionslösungen, Salben, Gele, topische Sprühlösungen, Pflaster, feste oder flüssige Inhalationsformulierungen und Suppositorien zusammen mit den üblichen pharmazeutischen Hilfsstoffen hergestellt werden.

## Claims

1. Use of the R-enantiomers of arylpropionic acids or of their pharmacologically compatible salts or derivatives, in pure form or, in comparison with the racemate, in enriched form for the production of medicaments for the treatment of chronically destructive rheumatic deseases of cartilages and joints.

2. Use according to claim 1, **characterised in that**, as R-arylpropionic acid compound, there are used R-flurbiprofen or its derivatives.

3. Use according to claim 1, **characterised in that** the compounds R-ketoprofen, R-naproxan, R-thioprofenic acid or R-fenoprofen, scarcely inverted in the case of humans, or pharmacologically compatible salts or derivatives of these compounds are used.

4. Use according to claim 1 to 3, **characterised in that** the active materials are used combined with basis therapeutics.

5. Use according to claim 1 to 4, **characterised in that** the individual dosings of the active materials lies between 30 mg and 1200 mg and the daily dosings between 60 mg and 3600 mg.

6. Use according to claim 1 to 5, **characterised in that** the R-arylpropionic acids are present in free form, as salt with inorganic or organic salt formers or complexes or as acid esters or acid amides.

7. Use according to claim 1 to 6, **characterised in that** the medicaments are produced as pharmaceutical formulations, such as tablets, capsules, dragees, drink granulates, drink tablets, drops, chewable formulations, injection solutions, ointments, gels, topical spray solutions, plasters, solid or liquid inhalation formulations and suppositories, together with the usual pharmaceutical adjuvants.

## Revendications

1. Utilisation de l'énantiomère R d'acides arylpropioniques ou de leurs sels ou dérivés pharmacologiquement acceptables, sous forme pure ou sous forme enrichie par rapport au racémate, pour la préparation de médicaments destinés au traitement de maladies rhumatismales chroniques-destructives du cartilage et des articulations.

2. Utilisation selon la revendication 1, **caractérisée en ce que** comme acide R-arylpropionique, on utilise le R-flurbiprofène ou ses dérivés.

3. Utilisation selon la revendication 1, **caractérisée en ce que** l'on utilise des composés qui ne s'inversent pratiquement pas chez l'homme, tels que le R-kétoprofène, le R-naproxène, l'acide R-thiaprofénique ou le R-fénoprofène, ou les sels ou dérivés pharmacologiquement acceptables de ces composés.

4. Utilisation selon l'une quelconque des revendications 1 à 3, **caractérisée en ce que** les principes actifs sont utilisés en combinaison avec un traitement de base.

5. Utilisation selon l'une quelconque des revendications 1 à 4, **caractérisée en ce que** la dose unitaire des principes actifs est comprise entre 30 mg et 1200 mg, et la posologie journalière, entre 60 mg et 3600 mg.

6. Utilisation selon l'une quelconque des revendications 1 à 5, **caractérisée en ce que** les acides R-arylpropioniques se trouvent sous forme libre, sous forme de sel formé avec des formateurs de sel inorganiques ou organiques ou sous forme de complexe, ou encore sous forme d'ester ou d'amide d'acide.

7. Utilisation selon l'une quelconque des revendications 1 à 6, **caractérisée en ce que** le médicament est présenté sous la forme de formulations pharmaceutiques, telles que des comprimés, des capsules, des dragées, des granulés buvables, des comprimés buvables, des gouttes, des formulations à mâcher, des solutions injectables, des onguents, des gels, des solutions pour pulvérisation locale, des emplâtres, des formulations à inhaler solides ou liquides et des suppositoires, conjointement avec des adjuvants pharmaceutiques usuels.
